(19)

Europäisches
Patentamt
European
Patent Office
Office européen
des brevets

(11) **EP 3 214 165 A1**

(12) **EUROPEAN PATENT APPLICATION**
published in accordance with Art. 153(4) EPC

(43) Date of publication:
**06.09.2017 Bulletin 2017/36**

(21) Application number: **15855508.6**

(22) Date of filing: **30.10.2015**

(51) Int Cl.:
*C12M 3/00* [(2006.01)]     *A61L 27/00* [(2006.01)]
*D01F 6/62* [(2006.01)]     *D03D 1/00* [(2006.01)]

(86) International application number:
**PCT/JP2015/080675**

(87) International publication number:
**WO 2016/068279 (06.05.2016 Gazette 2016/18)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA**

(30) Priority: **31.10.2014   JP 2014222967**

(71) Applicant: **Toray Industries, Inc.**
**Tokyo 103-8666 (JP)**

(72) Inventors:
• **KADOWAKI, Koji**
**Otsu-shi**
**Shiga 520-8558 (JP)**
• **KUGA, Chisa**
**Otsu-shi**
**Shiga 520-8558 (JP)**

• **FUJITA, Masaki**
**Otsu-shi**
**Shiga 520-8558 (JP)**
• **TANAHASHI, Kazuhiro**
**Otsu-shi**
**Shiga 520-8558 (JP)**
• **YAMADA, Satoshi**
**Otsu-shi**
**Shiga 520-2141 (JP)**
• **KANEKO, Takayuki**
**Otsu-shi**
**Shiga 520-2141 (JP)**
• **TSUCHIKURA, Hiroshi**
**Otsu-shi**
**Shiga 520-2141 (JP)**

(74) Representative: **Kador & Partner**
**Corneliusstraße 15**
**80469 München (DE)**

(54) **FIBER STRUCTURE FOR USE AS CELL SCAFFOLD MATERIAL**

(57)     The present invention aims to provide a fiber structure which can be used as a cell scaffold material showing improvement in both the cellular adhesiveness and the cell growth capacity, which improvement was achieved by controlling the fiber orientation in the multifilament and the average fiber diameter, which are physical properties. The present invention provides a fiber structure which can be used as a cell scaffold material, which fiber structure comprises a multifilament formed by bundling monofilaments having an average fiber diameter of 1 to 15 $\mu$m, wherein each of the monofilaments satisfies the condition of the following Formula 1: $(Y/X) \times 100 \geq 50$ ... Formula 1 [wherein in Formula 1, X represents the number of monofilaments for which the average crossing angle is investigated, and Y represents the number of monofilaments having an average crossing angle of not more than 25° in X].

**Description**

TECHNICAL FIELD

The present invention relates to a fiber structure which can be used as a cell scaffold material.

BACKGROUND ART

Base materials using various materials have been conventionally developed as base materials for supporting cells thereon during cell culture. For example, in general cell culture, surface-hydrophilized polystyrene (hereinafter referred to as "PS") plastic culture dishes and glass culture dishes are used. Cells are cultured by allowing their adhesion, spreading, and growth on these culture dishes.

On the other hand, it is known that, since the culture environment during culture on a plastic culture dish or a glass culture dish is largely different from an *in vivo* environment, material properties such as chemical properties, shape, and mechanical properties of the material surface may influence adhesion, spreading, growth, migration, and differentiation of cells during their culture. In view of this, studies have been carried out for development of cell scaffold materials using various materials for the purpose of controlling material properties such as chemical properties, shape, and mechanical properties of the material surface.

In particular, since the actual extracellular matrix is known to be constituted by micron-sized skeletons and nano-sized fibers, a number of cell scaffold materials using fiber structures such as non-woven fabrics, woven fabrics, and knitted fabrics as materials have been developed in order to mimic *in vivo* structures.

For example, in terms of the cell scaffold materials using non-woven fabrics, preparation of a non-woven fabric composed of polylactic acid using a method called the electrospinning method, wherein a solution containing a fiber-forming substance composed of a highly bioavailable polylactic acid is introduced into an electric field, and the liquid is drawn toward an electrode, thereby forming fibers, has been disclosed. It is reported that use of the electrospinning method enables preparation of a non-woven fabric having a smooth surface with a micron-sized fiber diameter, and that improvement of the cellular adhesiveness is possible by the micron-sized non-woven fabric (Patent Documents 1 and 2).

It is also reported that, when a non-woven fabric is prepared by the electrospinning method, uniformity of the surface properties, fiber diameter, and fiber orientation of the non-woven fabric influences the cellular adhesiveness and the like (Non-patent Document 1).

In terms of the cell scaffold materials using knitted fabrics, formation of a tubular body by alternatively knitting fiber bundles prepared by bundling a plurality of ultrafine fibers having a diameter of about 1 to 50 $\mu$m composed of polylactic acid, and use of the tubular body after coating of its outer surface with collagen as a scaffold for induction of regeneration and growth of nerve cells, have been reported (Patent Document 3).

In terms of the cell scaffold materials using woven fabrics, use of a woven fabric of polyester ultrafine fibers having a fiber fineness of not more than 1.0 denier as a cell scaffold material has been reported (Patent Document 4). It is reported that this woven fabric of ultrafine fibers is prepared by weaving composite fibers using polyester as an island component, and PS as a sea component, and then performing sea removal treatment to remove the sea component PS, thereby allowing formation of a woven fabric of polyester ultrafine fibers, followed by fluffing the tissue surface of the ultrafine fibers to increase the cell-contacting area, to thereby improve the cellular adhesiveness.

As a method for improving chemical properties of the material surface, a method in which the surface of ultrafine fibers of not more than 1.0 denier is subjected to plasma treatment, or sulfone groups and/or carboxyl groups are given to the surface to give anionic hydrophilic properties to the surface, to thereby increase the cell affinity and hence to improve the cell growth capacity (Patent Document 5).

PRIOR ART DOCUMENTS

[Patent Documents]

[Patent Document 1] JP 2004-290133 A
[Patent Document 2] JP 2012-192105 A
[Patent Document 3] JP 2009-153947 A
[Patent Document 4] WO 88/002398
[Patent Document 5] JP 01-034276 A

[Non-patent Document]

[Non-patent Document 1] A. Hadjizadeh et al., Journal of Biomedical Nanotechnology, 2013, vol. 9 (7), p. 1195

SUMMARY OF THE INVENTION

PROBLEMS TO BE SOLVED BY THE INVENTION

However, although Patent Documents 1 to 5 describe improvement of the cell culture efficiency, especially the adhesiveness, by changing physical properties of fibers such as the fiber diameter, or by changing chemical properties of the fiber surface, they do not describe improvement of the adhesiveness as well as the growth capacity of cells by controlling both the orientation of fibers constituting the fiber bundle and the average fiber diameter.
The cell culture base material used in Non-patent Document 1 is a non-woven fabric composed of monofilaments. Therefore, although its fiber diameter can be controlled, its fiber orientation cannot be sufficiently uniform.
Although Patent Document 4 describes improvement of the cellular adhesiveness by increasing the cell-contacting area by fluffing of the tissue surface of the ultrafine fibers, the orientation of the fibers constituting the fiber bundle cannot be uniform in cases where the fluffing is carried out.
Although there are methods, such as the method in Patent Document 5, in which the cell growth capacity is improved by chemical modification of the fiber surface, an additional process is required for the chemical modification in cases where chemical modification and the like are carried out, which is problematic.
In view of this, an object of the present invention is to provide a fiber structure which can be used as a cell scaffold material showing improvement in both the cellular adhesiveness and the cell growth capacity, which improvement is achieved by controlling the fiber orientation in the multifilament and the average fiber diameter, which are physical properties.

MEANS FOR SOLVING THE PROBLEMS

As a result of intensive study to solve the problems described above, the present inventors discovered the following inventions (1) to (7).

(1) A fiber structure which can be used as a cell scaffold material, the fiber structure comprising a multifilament formed by bundling monofilaments having an average fiber diameter of 1 to 15 $\mu$m, wherein each monofilament in the multifilament satisfies the condition of the following Formula 1:

$$(Y/X) \times 100 \geq 50 \ \ldots \text{ Formula 1}$$

[wherein in Formula 1, X represents the number of monofilaments for which the average crossing angle is investigated, and Y represents the number of monofilaments having an average crossing angle of not more than 25° in X].
(2) The fiber structure according to (1), which is a woven fabric.
(3) The fiber structure according to (1) or (2), wherein the monofilament arranged on the surface of the multifilament is a monofilament containing a polymer selected from the group consisting of polyester, polypropylene, acryl, polyamide, polystyrene, polyvinyl chloride, polyurethane, polysulfone, polyethersulfone, and polymethyl methacrylate.
(4) The fiber structure according to (3), wherein the monofilament arranged on the surface of the multifilament is a monofilament composed of polyethylene terephthalate or polybutylene terephthalate.
(5) The fiber structure according to any one of (1) to (4), wherein the cross-sectional shape of the monofilament is a flat multilobed shape with six to ten lobes.
(6) A cell scaffold comprising the fiber structure according to any one of (1) to (5).
(7) A cell scaffold for medical use, comprising the fiber structure according to any one of (1) to (5).

EFFECT OF THE INVENTION

The fiber structure which can be used as a cell scaffold material of the present invention improves both the cellular adhesiveness and the cell growth capacity since both the orientation of monofilaments in the multifilament and the average fiber diameter are controlled, so that use of the fiber structure as an excellent cell scaffold material is possible.

MODE FOR CARRYING OUT THE INVENTION

The fiber structure which can be used as a cell scaffold material of the present invention is characterized in that it comprises a multifilament formed by bundling monofilaments having an average fiber diameter of 1 to 15 $\mu$m, wherein each monofilament in the multifilament satisfies the condition of the following Formula 1:

$$(Y/X)\times100\geq50 \ldots \text{Formula 1}$$

[wherein in Formula 1, X represents the number of monofilaments for which the average crossing angle is investigated, and Y represents the number of monofilaments having an average crossing angle of not more than 25° in X].
Modes for carrying out the present invention are described below, but the present invention is not limited to these modes. The following terms used in the present description are defined as described below unless otherwise specified.
"Cell scaffold" means a base material used for culturing cells *in vivo* or *in vitro,* and "cell scaffold material" means a material to be used as a cell scaffold.
The cell scaffold material of the present invention may be used for culturing any cells *in vivo* or *in vitro.* The cell scaffold material of the present invention is preferably used for culturing adherent cells from the viewpoint of better exertion of the action to immobilize cells by adhesion.
"Multifilament" means a fiber bundle formed by bundling a plurality of monofilaments, and "monofilaments having an average crossing angle of not more than 25°"" means monofilaments constituting a multifilament that are crossed with each other and have an average crossing angle S of not more than 25°, or monofilaments constituting a multifilament that are not crossed with each other (average crossing angle S = 0°).
"Average crossing angle S" means a value determined by arbitrarily selecting a multifilament from a fiber structure, focusing on positions where monofilaments in the multifilament are crossed with their adjacent monofilaments based on observation of a photograph at a magnification of $\times$ 400 (viewing area, about 0.48 mm$^2$), choosing the three positions having the largest crossing angles, and calculating the average value of the three crossing angles. Two angles are formed when two monofilaments are crossed with each other. The crossing angle corresponds to the smaller angle, that is, the angle having a value of 0° to 90°. In cases where no position in a multifilament is found to have a crossing angle of not less than 25°, the monofilaments constituting the multifilament are regarded as being not crossed with each other (average crossing angle S = 0°).
In cases where the average crossing angle S between the monofilaments constituting the multifilament is not less than 25°, the monofilaments have different orientations, so that the cellular adhesiveness and the cell growth capacity decrease. For achievement of a uniform monofilament orientation, the woven fabric is preferably produced such that the fiber direction is not disturbed by, for example, yarn breakage or fluffing in the multifilament, and such that steps by application of an external force such as fabric raising, loop formation, and water jet punching to the multifilament portion are avoided. The average crossing angle S is most preferably 0° from the viewpoint of the monofilament orientation.
The ratio of monofilaments having an average crossing angle S of not more than 25° in the multifilament is calculated according to the following Formula 1. A sample was equally divided into four portions such that the angle of the intersection was 90°, and the average crossing angle was measured for 10 monofilaments in each portion (a total of 40 monofilaments) (the measurement was carried out for three positions per monofilament, that is, a total of 120 positions). The ratio was calculated according to the following Formula 1.

$$(Y/X)\times100\geq50 \ldots \text{Formula 1}$$

[wherein in Formula 1, X represents the number of monofilaments for which the average crossing angle was investigated, and Y represents the number of monofilaments having an average crossing angle of not more than 25° in X].
In the above Formula 1, the value of $(Y/X)\times100$ is preferably not less than 50, more preferably 100. In cases where the value of $(Y/X)\times100$ is less than 50, inhibition of the growth of cells along the orientation occurs, leading to a decrease in the cell growth capacity, which is not preferred.
"Average fiber diameter" is a value determined by observing cross sections of monofilaments in the multifilament at arbitrary 10 positions using a scanning electron microscope (manufactured by Hitachi High-Technologies Corporation), and calculating the average of their diameters. In cases where the monofilament has a flat six- to ten-lobed shape, "average fiber diameter" is a value calculated by averaging the long diameter A and the short diameter B, wherein the long diameter A is the longest diameter connecting apexes on the circumcircle of the flat multilobed shape, and the short diameter B is the longest short diameter among the short diameters corresponding to the diameters connecting the apexes of the protruded portions of the flat multilobed shape.

The average fiber diameter of the multifilament is preferably 1 to 15 $\mu$m, more preferably 1 to 10 $\mu$m. In cases where the average fiber diameter is not more than 1 $\mu$m or not less than 10 $\mu$m, the cellular adhesiveness decreases, which is not preferred.

Preferred specific examples of the fiber structure include non-woven fabrics, woven fabrics, knitted fabrics, tubes, and meshes. Woven fabrics are more preferred.

Although the multifilament may be used for one of the warp and the weft in the woven fabric, the multifilament is preferably used for both the warp and the weft. The "surface of the multifilament" is composed of the monofilaments exposed on the surface among the monofilaments forming the multifilament. The "surface of the multifilament" also includes monofilaments only partially exposed on the surface.

Although the type of the monofilaments constituting the multifilament is not limited, the monofilaments arranged on the surface of the multifilament are preferably monofilaments composed of a polymer selected from the group consisting of polyester, polypropylene, nylon, acryl, polyamide, and PS. From the viewpoint of the cost, cellular adhesiveness, and cell growth capacity, the monofilaments are preferably those composed of polyester.

The monofilaments composed of polyester are preferably monofilaments composed of polyethylene terephthalate, polybutylene terephthalate, or nylon. The monofilaments are more preferably composed of polyethylene terephthalate or polybutylene terephthalate.

The cross-sectional shape of the monofilament constituting the multifilament is not limited. Examples of the cross-sectional shape include known cross-sectional shapes such as circular, triangular, flat, and hollow shapes. The cross-sectional shape is preferably a flat multilobed shape with six to ten lobes, more preferably a flat eight-lobed shape.

"The cross-sectional shape of the monofilament is a flat multilobed shape with six to ten lobes" means that the monofilament has a cross-sectional shape which satisfies the following Formulae 2 to 5 at the same time:

$$\text{Degree of flatness (A/B)} = 1.2 \text{ to } 2.2 \ldots \text{Formula 2}$$

$$\text{Degree of deformation I (C/D)} = 1.1 \text{ to } 1.3 \ldots \text{Formula 3}$$

$$\text{Degree of flatness II (B/D)} = 1.1 \text{ to } 1.6 \ldots \text{Formula 4}$$

$$A > B \geq C > D \ldots \text{Formula 5}$$

wherein A represents the long diameter A, which is the longest diameter connecting apexes on the circumcircle of the flat multilobed shape; B represents the short diameter B, which is the longest short diameter perpendicular to the long diameter A among the short diameters that correspond to the diameters connecting the apexes of the protruded portions of the flat multilobed shape; C represents the short diameter C, which is the same as the short diameter B or the second longest short diameter; and D represents the short diameter D, which is the shortest short diameter among the short diameters connecting the bottom points of the recessed portions of the flat multilobed shape.

The fiber structure is preferably used *in vitro* as a cell scaffold. More specifically, the fiber structure is preferably used as a base material for use in culture of cells *in vitro.*

The fiber structure is preferably used as a cell scaffold for medical use. More specifically, the fiber structure is more preferably used for medical equipments for implanting to be embedded in the body, such as artificial blood vessels and stent-grafts.

EXAMPLES

The present invention is described below in detail by way of Examples and Comparative Examples. However, the present invention is not limited thereto. The monofilament fineness in each of Examples and Comparative Examples is calculated according to the procedure of JIS L 1013 (2010) 8.3.1 A, wherein the fineness based on the corrected weight is measured at a predetermined load of 0.045 cN/dtex to provide the total fineness, and the resulting total fineness is divided by the number of monofilaments.

(Example 1)

A woven fabric constituted by warp and weft yarns composed of multifilaments having a monofilament fineness of about 2.33 dtex and a total fineness of 84 dtex, wherein each multifilament is constituted by monofilaments composed of polyester fibers and having a circular cross-sectional shape, was prepared to provide a fiber structure 1. As a result of evaluation of the average fiber diameter using a scanning electron microscope (manufactured by Hitachi High-Technologies Corporation), the average fiber diameter was found to be 15 μm. As a result of measurement of the average crossing angle S using a microscope VHX-2000 (manufactured by Keyence Corporation), the value of the above Formula 1, $(Y/X) \times 100$, was found to be 100 in the fiber structure 1. The fiber structure 1 prepared was sterilized with ethylene oxide gas (hereinafter referred to as "EOG"), and subjected to tests for the cell growth capacity and the cellular adhesiveness. The results are shown in Table 1.

(Example 2)

A woven fabric constituted by warp and weft yarns composed of multifilaments having a monofilament fineness of about 0.306 dtex and a total fineness of 44 dtex, wherein each multifilament is constituted by monofilaments composed of polyester fibers and having a circular cross-sectional shape, was prepared to provide a fiber structure 2. As a result of evaluation of the average fiber diameter using a scanning electron microscope (manufactured by Hitachi High-Technologies Corporation), the average fiber diameter was found to be 5 μm. As a result of measurement of the average crossing angle S using a microscope VHX-2000 (manufactured by Keyence Corporation), the value of the above Formula 1, $(Y/X) \times 100$, was found to be 100 in the fiber structure 2. The fiber structure 2 prepared was sterilized with EOG, and subjected to tests for the cell growth capacity and the cellular adhesiveness. The results are shown in Table 1.

(Example 3)

A woven fabric constituted by warp and weft yarns composed of multifilaments having a monofilament fineness of about 0.0838 dtex and a total fineness of 52.8 dtex, wherein each multifilament is constituted by monofilaments composed of polyester fibers and having a circular cross-sectional shape, was prepared to provide a fiber structure 3. As a result of evaluation of the average fiber diameter using a scanning electron microscope (manufactured by Hitachi High-Technologies Corporation), the average fiber diameter was found to be 3 μm. As a result of measurement of the average crossing angle S using a microscope VHX-2000 (manufactured by Keyence Corporation), the value of the above Formula 1, $(Y/X) \times 100$, was found to be 100 in the fiber structure 3. The fiber structure 3 prepared was sterilized with EOG, and subjected to tests for the cell growth capacity and the cellular adhesiveness. The results are shown in Table 1.

(Example 4)

A woven fabric constituted by warp and weft yarns composed of multifilaments having a monofilament fineness of about 0.0125 dtex and a total fineness of 56 dtex, wherein each multifilament is constituted by monofilaments composed of polyester fibers and having a circular cross-sectional shape, was prepared to provide a fiber structure 4. As a result of evaluation of the average fiber diameter using a scanning electron microscope (manufactured by Hitachi High-Technologies Corporation), the average fiber diameter was found to be 1 μm. As a result of measurement of the average crossing angle S using a microscope VHX-2000 (manufactured by Keyence Corporation), the value of the above Formula 1, $(Y/X) \times 100$, was found to be 100 in the fiber structure 4. The fiber structure 4 prepared was sterilized with EOG, and subjected to tests for the cell growth capacity and the cellular adhesiveness. The results are shown in Table 1.

(Example 5)

A woven fabric constituted by warp and weft yarns composed of multifilaments having a monofilament fineness of about 0.0838 dtex and a total fineness of 52.8 dtex, wherein each multifilament is constituted by monofilaments composed of polyester fibers and having a circular cross-sectional shape, was prepared. As a result of evaluation of the average fiber diameter using a scanning electron microscope (manufactured by Hitachi High-Technologies Corporation), the average fiber diameter was found to be 3 μm. A half portion of the prepared woven fabric was fluffed using abrasive paper, and the border section was punched out using a puncher such that the value of the above Formula 1, $(Y/X) \times 100$, became 50, to provide a fiber structure 5. As a result of measurement of the average crossing angle S using a microscope VHX-2000 (manufactured by Keyence Corporation), the value of the above Formula 1, $(Y/X) \times 100$, was found to be 50 in the fiber structure 5. The fiber structure 5 prepared was sterilized with EOG, and subjected to tests for the cell growth capacity and the cellular adhesiveness. The results are shown in Table 1.

(Example 6)

A woven fabric constituted by warp and weft yarns composed of multifilaments having a monofilament fineness of about 1.56 dtex and a total fineness of 56 dtex, wherein each multifilament is constituted by monofilaments composed of polyester fibers and having a flat eight-lobed cross-sectional shape, was prepared to provide a fiber structure 6. As a result of evaluation of the average fiber diameter using a scanning electron microscope (manufactured by Hitachi High-Technologies Corporation), the average fiber diameter was found to be 12 μm. As a result of measurement of the average crossing angle S using a microscope VHX-2000 (manufactured by Keyence Corporation), the value of the above Formula 1, (Y/X)×100, was found to be 100 in the fiber structure 6. The fiber structure 6 prepared was sterilized with EOG, and subjected to tests for the cell growth capacity and the cellular adhesiveness. The results are shown in Table 1.

(Comparative Example 1)

A woven fabric constituted by warp and weft yarns composed of multifilaments having a monofilament fineness of about 5.6 dtex and a total fineness of 84 dtex, wherein each multifilament is constituted by monofilaments composed of polyester fibers and having a circular cross-sectional shape, was prepared to provide a fiber structure 7. As a result of evaluation of the average fiber diameter using a scanning electron microscope (manufactured by Hitachi High-Technologies Corporation), the average fiber diameter was found to be 23 μm. As a result of measurement of the average crossing angle S using a microscope VHX-2000 (manufactured by Keyence Corporation), the value of the above Formula 1, (Y/X)×100, was found to be 100 in the fiber structure 7. The fiber structure 7 prepared was sterilized with EOG, and subjected to tests for the cell growth capacity and the cellular adhesiveness. The results are shown in Table 1.

(Comparative Example 2)

A woven fabric constituted by warp and weft yarns composed of multifilaments having a monofilament fineness of about 0.00625 dtex and a total fineness of 56 dtex, wherein each multifilament is constituted by monofilaments composed of polyester fibers and having a circular cross-sectional shape, was prepared to provide a fiber structure 8. As a result of evaluation of the average fiber diameter using a scanning electron microscope (manufactured by Hitachi High-Technologies Corporation), the average fiber diameter was found to be 0.7 μm. As a result of measurement of the average crossing angle S using a microscope VHX-2000 (manufactured by Keyence Corporation), the value of the above Formula 1, (Y/X)×100, was found to be 100 in the fiber structure 8. The fiber structure 8 prepared was sterilized with EOG, and subjected to tests for the cell growth capacity and the cellular adhesiveness. The results are shown in Table 1.

(Comparative Example 3)

A woven fabric constituted by warp and weft yarns composed of multifilaments having a monofilament fineness of about 0.0838 dtex and a total fineness of 52.8 dtex, wherein each multifilament is constituted by monofilaments composed of polyester fibers and having a circular cross-sectional shape, was prepared. As a result of evaluation of the average fiber diameter using a scanning electron microscope (manufactured by Hitachi High-Technologies Corporation), the average fiber diameter was found to be 3 μm. A three-quarter portion of the prepared woven fabric was fluffed using abrasive paper, and the border section was punched out using a puncher such that the value of the above Formula 1, (Y/X)×100, became 25, to provide a fiber structure 9. As a result of measurement of the average crossing angle S using a microscope VHX-2000 (manufactured by Keyence Corporation), the value of the above Formula 1, (Y/X)×100, was found to be 25 in the fiber structure 9. The fiber structure 9 prepared was sterilized with EOG, and subjected to tests for the cell growth capacity and the cellular adhesiveness. The results are shown in Table 1.

(Comparative Example 4)

A woven fabric constituted by warp and weft yarns composed of multifilaments having a monofilament fineness of about 0.0838 dtex and a total fineness of 52.8 dtex, wherein each multifilament is constituted by monofilaments composed of polyester fibers and having a circular cross-sectional shape, was prepared. As a result of evaluation of the average fiber diameter using a scanning electron microscope (manufactured by Hitachi High-Technologies Corporation), the average fiber diameter was found to be 3 μm. The whole portion of the prepared woven fabric was fluffed using abrasive paper, and punched out using a puncher such that the value of the above Formula 1, (Y/X)×100, became 0, to provide a fiber structure 10. As a result of measurement of the average crossing angle S using a microscope VHX-2000 (manufactured by Keyence Corporation), the value of the above Formula 1, (Y/X)×100, was found to be 0 in the fiber structure 10. The fiber structure 10 prepared was sterilized with EOG, and subjected to tests for the cell growth capacity and the cellular adhesiveness. The results are shown in Table 1.

(Comparative Example 5)

A woven fabric constituted by warp and weft yarns composed of multifilaments having a monofilament fineness of about 0.0125 dtex and a total fineness of 56 dtex, wherein each multifilament is constituted by monofilaments composed of polyester fibers and having a circular cross-sectional shape, was prepared. As a result of evaluation of the average fiber diameter using a scanning electron microscope (manufactured by Hitachi High-Technologies Corporation), the average fiber diameter was found to be 1 $\mu$m. The whole portion of the prepared woven fabric was fluffed using abrasive paper, and punched out using a puncher such that the value of the above Formula 1, (Y/X)$\times$100, became 0, to provide a fiber structure 11. As a result of measurement of the average crossing angle S using a microscope VHX-2000 (manufactured by Keyence Corporation), the value of the above Formula 1, (Y/X)$\times$100, was found to be 0 in the fiber structure 11. The fiber structure 11 prepared was sterilized with EOG, and subjected to tests for the cell growth capacity and the cellular adhesiveness. The results are shown in Table 1.

(Comparative Example 6)

Polyethylene terephthalate pellets were dissolved in the mixed solvent of 1:1 of dichloromethane (Wako Pure Chemical Industries, Ltd.) and trifluoroacetic acid (Wako Pure Chemical Industries, Ltd.) for 24 hours with stirring. Using an electrospinning device, a non-woven fabric having an average fiber diameter of 2 $\mu$m was prepared to provide a fiber structure 12. As a result of evaluation of the average fiber diameter using a scanning electron microscope (manufactured by Hitachi High-Technologies Corporation), the average fiber diameter was found to be 2 $\mu$m. As a result of measurement of the average crossing angle S using a microscope VHX-2000 (manufactured by Keyence Corporation), the value of the above Formula 1, (Y/X)$\times$100, was found to be 0 in the fiber structure 12. The fiber structure 12 was sterilized with EOG, and subjected to tests for the cell growth capacity and the cellular adhesiveness. The results are shown in Table 1.

(Example 7)

A woven fabric constituted by warp and weft yarns composed of multifilaments having a monofilament fineness of about 1.56 dtex and a total fineness of 56 dtex, wherein each multifilament is constituted by monofilaments composed of polyester fibers and having a flat six-lobed cross-sectional shape, was prepared to provide a fiber structure 13. As a result of evaluation of the average fiber diameter using a scanning electron microscope (manufactured by Hitachi High-Technologies Corporation), the average fiber diameter was found to be 12 $\mu$m. As a result of measurement of the average crossing angle S using a microscope VHX-2000 (manufactured by Keyence Corporation), the value of the above Formula 1, (Y/X)$\times$100, was found to be 100 in the fiber structure 13. The fiber structure 13 prepared was sterilized with EOG, and subjected to tests for the cell growth capacity and the cellular adhesiveness. The results are shown in Table 1.

(Example 8)

A woven fabric constituted by warp and weft yarns composed of multifilaments having a monofilament fineness of about 1.56 dtex and a total fineness of 56 dtex, wherein each multifilament is constituted by monofilaments composed of polyester fibers and having a flat ten-lobed cross-sectional shape, was prepared to provide a fiber structure 14. As a result of evaluation of the average fiber diameter using a scanning electron microscope (manufactured by Hitachi High-Technologies Corporation), the average fiber diameter was found to be 12 $\mu$m. As a result of measurement of the average crossing angle S using a microscope VHX-2000 (manufactured by Keyence Corporation), the value of the above Formula 1, (Y/X)$\times$100, was found to be 100 in the fiber structure 14. The fiber structure 14 prepared was sterilized with EOG, and subjected to tests for the cell growth capacity and the cellular adhesiveness. The results are shown in Table 1.

(Comparative Example 7)

A woven fabric constituted by warp and weft yarns composed of multifilaments having a monofilament fineness of about 0.00114 dtex and a total fineness of 70 dtex, wherein each multifilament is constituted by monofilaments composed of polyester fibers and having a circular cross-sectional shape, was prepared to provide a fiber structure 15. As a result of evaluation of the average fiber diameter using a scanning electron microscope (manufactured by Hitachi High-Technologies Corporation), the average fiber diameter was found to be 0.3 $\mu$m. As a result of measurement of the average crossing angle S using a microscope VHX-2000 (manufactured by Keyence Corporation), the value of the above Formula 1, (Y/X)$\times$100, was found to be 100 in the fiber structure 11. The fiber structure 15 prepared was sterilized with EOG, and subjected to tests for the cell growth capacity and the cellular adhesiveness. The results are shown in Table 1.

(Example 9)

The fiber structure 2 prepared in Example 2 was used after sterilization with EOG, and the cell growth rate was evaluated. The results are shown in Table 2.

(Example 10)

The fiber structure 3 prepared in Example 3 was used after sterilization with EOG, and the cell growth rate was evaluated. The results are shown in Table 2.

(Example 11)

The fiber structure 7 prepared in Example 13 was used after sterilization with EOG, and the cell growth rate was evaluated. The results are shown in Table 2.

(Comparative Example 8)

The fiber structure 8 prepared in Comparative Example 2 was used after sterilization with EOG, and the cell growth rate was evaluated. The results are shown in Table 2.

(Comparative Example 9)

The fiber structure 10 prepared in Comparative Example 4 was used after sterilization with EOG, and the cell growth rate was evaluated. The results are shown in Table 2.

(Evaluation 1: Cell Growth Capacity/Cellular Adhesiveness Test)

Each of the fiber structures 1 to 12 was punched into a disk sample having a diameter of 15 mm using a puncher. Each disk sample was placed in a well of a 24-well microplate for cell culture (manufactured by Sumitomo Bakelite Co., Ltd.) such that the inner-wall side faced upward, and a metal pipe-shaped weight having a thickness of 1 mm was placed on the top of the sample. To each well, normal human umbilical vein endothelial cells (Takara Bio Inc.) suspended in 2% FBS endothelial cell culture kit-2 (manufactured by Takara Bio Inc.) were added such that the well contained $5 \times 10^4$ cells. The cells were cultured in 1 mL of a medium at 37°C under an environment of 5% $CO_2$ for 48 hours. After rinsing the well with PBS(-) (manufactured by Nissui Pharmaceutical Co., Ltd.), 1 mL of a medium was added thereto, followed by addition of 100 μL of Cell Counting Kit-8 (manufactured by Dojindo Laboratories). The cells were then cultured at 37°C under an environment of 5% $CO_2$ for 4 hours. Subsequently, the absorbance at 450 nm was measured using a microplate reader (MTP-300, manufactured by Corona Electric Co., Ltd.), followed by calculation of the absorbance as shown by the following Formula 6.

$$As = At - Ab \ldots \text{Formula 6}$$

At: measured absorbance
Ab: absorbance of the blank solution (the medium, and the solution of Cell Counting Kit-8; containing no cells)
As: calculated absorbance

Here, since the amount of grown cells after the culture can be known from the calculated absorbance As, a score for the cell growth was determined based on the absorbance As. More specifically, in cases where As was less than 0.3, the cell growth capacity was judged as being weak (+); in cases where As was not less than 0.3 and less than 0.5, the cell growth capacity was judged as being moderate (++); and, in cases where As was not less than 0.5, the cell growth capacity was judged as being strong (+++).
After fixing the cells in 10% formalin solution (manufactured by Wako Pure Chemical Industries, Ltd.), the shapes of adherent cells were observed using a scanning electron microscope (manufactured by Hitachi High-Technologies Corporation). The sample was equally divided into four portions, and observation of surfaces was carried out for each portion at a magnification of ×1000. The length of the minor axis and the length of the major axis were measured for not less than 30 cells in a total of 16 viewing areas where two or more cells are present, corresponding to 4 different viewing areas in each portion. According to the following Formula 7, the aspect ratio of each cell was calculated.

$$Ls = La/Lb \ldots \text{Formula 7}$$

La: length of the cell in the direction of the major axis
Lb: length of the cell in the direction of the minor axis
Ls: calculated aspect ratio of the cell

Since adhesion of the cell can be judged based on the thus calculated aspect ratio Ls, the ratio of adherent cells to the observed cells, Rs, was calculated according to the following Formula 8. A score for the cell adhesiveness was then determined based on the calculated value. More specifically, in cases where Rs was not more than 50, the cellular adhesiveness was judged as being weak (+); in cases where Rs was more than 25 and less than 50, the cellular adhesiveness was judged as being moderate (++); and, in cases where Rs was not more than 25, the cellular adhesiveness was judged as being strong (+++).

$$Rs\ (\%) = (\text{number of cells satisfying } Ls \leq 2)\ /\ (\text{number of cells observed}) \times 100$$

$$\ldots \text{Formula 8}$$

(Evaluation 2: Evaluation of Cell Growth Rate)

Each of the fiber structures 2, 3, and 13 of Examples, and the fiber structures 8 and 10 of Comparative Examples, was punched into a disk sample having a diameter of 15 mm using a puncher. Each disk sample was placed in a well of a 24-well microplate for cell culture (manufactured by Sumitomo Bakelite Co., Ltd.) such that the inner-wall side faced upward, and a metal pipe-shaped weight having a thickness of 1 mm was placed on the top of the sample. NIH3T3 cells suspended in Iscove's modified Dulbecco's medium (manufactured by Sigma-Aldrich) supplemented with 10% FBS were added to the well at $4 \times 10^4$ cells/well. The cells were cultured in 1 mL of a medium at 37°C under an environment of 5% $CO_2$ for 24, 48, or 72 hours. After rinsing with PBS(-) (manufactured by Nissui Pharmaceutical Co., Ltd.), 500 μL of a medium was added to the cells, and 20 μL of Cell Counting Kit-8 (manufactured by Dojindo Laboratories) was then added thereto, followed by performing culture at 37°C under an environment of 5% $CO_2$ for 1 hour. Subsequently, the absorbance at 450 nm was measured using a microplate reader (MTP-300, manufactured by Corona Electric Co., Ltd.). The cell growth rates at Hour 48 and Hour 72 were calculated using the Formula 9 and the Formula 10 described below.

$$P_{48} = (A_{48} - Ab)\ /\ (A_{24} - Ab)\ \ldots \text{Formula 9}$$

$P_{48}$: cell growth rate at Hour 48
$A_{48}$: measured value of the absorbance at Hour 48 of the culture
$A_{24}$: measured value of the absorbance at Hour 24 of the culture
Ab: absorbance of the blank solution (the medium, and the solution of Cell Counting Kit-8; containing no cells)

$$P_{72} = (A_{72} - Ab)\ /\ (A_{24} - Ab)\ \ldots \text{Formula 10}$$

$P_{72}$: cell growth rate at Hour 72
$A_{72}$: measured value of the absorbance at Hour 72 of the culture
$A_{24}$: measured value of the absorbance at Hour 24 of the culture
Ab: absorbance of the blank solution (the medium, and the solution of Cell Counting Kit-8; containing no cells)

From the data shown in Table 1 and Table 2, it is clear that both the cell adhesiveness and the cell growth capacity can be improved by controlling the orientation of monofilaments in the multifilament and the average fiber diameter, which are physical properties.

[Table 1]

| | (X/Y)×100 | Average fiber diameter ($\mu$m) | Fiber structure | Fiber processing method | Cross-sectional shape of monofilament | Result of evaluation of cells | |
|---|---|---|---|---|---|---|---|
| | | | | | | Cell growth capacity | Cellular adhesiveness |
| Example 1 | 100 | 15 | Multifilament | Woven fabric | Circular shape | +++ | ++ |
| Example 2 | 100 | 5 | Multifilament | Woven fabric | Circular shape | +++ | +++ |
| Example 3 | 100 | 3 | Multifilament | Woven fabric | Circular shape | +++ | +++ |
| Example 4 | 100 | 1 | Multifilament | Woven fabric | Circular shape | +++ | +++ |
| Example 5 | 50 | 3 | Multifilament | Woven fabric | Circular shape | +++ | ++ |
| Example 6 | 100 | 12 | Multifilament | Woven fabric | Flat eight-lobed shape | +++ | +++ |
| Comparative Example 1 | 100 | 23 | Multifilament | Woven fabric | Circular shape | ++ | ++ |
| Comparative Example 2 | 100 | 0.7 | Multifilament | Woven fabric | Circular shape | ++ | ++ |
| Comparative Example 3 | 25 | 3 | Multifilament | Woven fabric | Circular shape | ++ | + |
| Comparative Example 4 | 0 | 3 | Multifilament | Woven fabric | Circular shape | ++ | + |
| Comparative Example 5 | 0 | 1 | Multifilament | Woven fabric | Circular shape | + | + |
| Comparative Example 6 | 0 | 2 | Monofilament | Non-woven fabric | Circular shape | ++ | + |
| Example 7 | 100 | 12 | Multifilament | Woven fabric | Flat six-lobed shape | +++ | +++ |
| Example 8 | 100 | 12 | Multifilament | Woven fabric | Flat ten-lobed shape | +++ | +++ |
| Comparative Example 7 | 100 | 0.3 | Multifilament | Woven fabric | Circular shape | + | + |

[Table 2]

| | (X/Y)×100 | Average fiber diameter (μm) | Fiber structure | Fiber processing method | Cross-sectional shape of monofilament | Cell growth rate | |
|---|---|---|---|---|---|---|---|
| | | | | | | At Hour 48 | At Hour 72 |
| Example 9 | 100 | 5 | Multifilament | Woven fabric | Circular shape | 1.5 | 2.2 |
| Example 10 | 100 | 3 | Multifilament | Woven fabric | Circular shape | 1.7 | 2.7 |
| Example 11 | 100 | 12 | Multifilament | Woven fabric | Flat eight-lobed shape | 1.9 | 3.1 |
| Comparative Example 8 | 100 | 0.7 | Multifilament | Woven fabric | Circular shape | 1.3 | 1.8 |
| Comparative Example 9 | 0 | 3 | Multifilament | Woven fabric | Circular shape | 1.2 | 1.4 |

INDUSTRIAL APPLICABILITY

The fiber structure which can be used as a cell scaffold material of the present invention can be used as a cell scaffold material excellent in the cell adhesiveness and the cell growth capacity. The fiber structure of the present invention can also be used by inclusion in a cell scaffold for medical use, especially for artificial blood vessels, stent-grafts, and the like.

**Claims**

1.  A fiber structure which can be used as a cell scaffold material, said fiber structure comprising a multifilament formed by bundling monofilaments having an average fiber diameter of 1 to 15 μm,
    wherein each monofilament in said multifilament satisfies the condition of the following Formula 1:

$$(Y/X)\times100\geq50 \ldots \text{Formula 1}$$

    [wherein in Formula 1, X represents the number of monofilaments for which the average crossing angle is investigated, and Y represents the number of monofilaments having an average crossing angle of not more than 25° in X].

2.  The fiber structure according to claim 1, which is a woven fabric.

3.  The fiber structure according to claim 1 or 2, wherein said monofilament is a monofilament containing a polymer selected from the group consisting of polyester, polypropylene, acryl, polyamide, polystyrene, polyvinyl chloride, polyurethane, polysulfone, polyethersulfone, and polymethyl methacrylate.

4.  The fiber structure according to claim 3, wherein said monofilament is a monofilament composed of polyethylene terephthalate or polybutylene terephthalate.

5.  The fiber structure according to any one of claims 1 to 4, wherein the cross-sectional shape of said monofilament is a flat multilobed shape with six to ten lobes.

6.  A cell scaffold comprising the fiber structure according to any one of claims 1 to 5.

7.  A cell scaffold for medical use, comprising the fiber structure according to any one of claims 1 to 5.

## INTERNATIONAL SEARCH REPORT

| International application No. |
| --- |
| PCT/JP2015/080675 |

### A. CLASSIFICATION OF SUBJECT MATTER
*C12M3/00*(2006.01)i, *A61L27/00*(2006.01)i, *D01F6/62*(2006.01)i, *D03D1/00* (2006.01)i

According to International Patent Classification (IPC) or to both national classification and IPC

### B. FIELDS SEARCHED

Minimum documentation searched (classification system followed by classification symbols)
C12M3/00, A61L27/00, D01F6/62, D03D1/00

Documentation searched other than minimum documentation to the extent that such documents are included in the fields searched
Jitsuyo Shinan Koho          1922–1996   Jitsuyo Shinan Toroku Koho   1996–2016
Kokai Jitsuyo Shinan Koho   1971–2016   Toroku Jitsuyo Shinan Koho   1994–2016

Electronic data base consulted during the international search (name of data base and, where practicable, search terms used)
JSTPlus/JMEDPlus/JST7580(JDreamIII), CAplus/MEDLINE/EMBASE/BIOSIS(STN)

### C. DOCUMENTS CONSIDERED TO BE RELEVANT

| Category* | Citation of document, with indication, where appropriate, of the relevant passages | Relevant to claim No. |
| --- | --- | --- |
| X<br>Y<br>A | WEN, Xuejun et al., Effect of filament diameter and extracellular matrix molecule precoating on neurite outgrowth and Schwann cell behavior on multifilament entubulation bridging device in vitro, J Biomed Mater Res Pt A, 2006.03.01, Vol.76, No.3, Page.626-637, page 626, Abstract, right column, lines 3 to 6, 11 to 13, page 635, right column, 7th to 2nd lines from the bottom, page 636, left column, line 6 to the last line, fig. 1, 5 | 1,3,4,6,7<br>5<br>2 |
| Y | WO 88/02398 A1 (Toray Industries, Inc.), 07 April 1988 (07.04.1988), claims 1, 2, 4 to 6; example 1<br>& US 5254471 A<br>claims 1 to 9, 11, 12; example 1<br>& EP 286688 A1        & DE 3784239 A | 1-7 |

☒ Further documents are listed in the continuation of Box C.          ☐ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 05 January 2016 (05.01.16) | 19 January 2016 (19.01.16) |

| Name and mailing address of the ISA/ | Authorized officer |
| --- | --- |
| Japan Patent Office<br>3-4-3,Kasumigaseki,Chiyoda-ku,<br>Tokyo 100-8915,Japan | Telephone No. |

Form PCT/ISA/210 (second sheet) (January 2015)

## INTERNATIONAL SEARCH REPORT

International application No.

PCT/JP2015/080675

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2004-148014 A (Nipro Corp.),<br>27 May 2004 (27.05.2004),<br>paragraph [0001]<br>& US 2006/0029639 A1<br>paragraph [0001]<br>& WO 2004/039578 A1 & EP 1577083 A1<br>& CN 1705559 A & AU 2003280598 A | 1-7 |
| Y | JP 2005-350777 A (Toray Industries, Inc.),<br>22 December 2005 (22.12.2005),<br>fig. 1<br>(Family: none) | 5 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004290133 A
- JP 2012192105 A
- JP 2009153947 A
- WO 88002398 A
- JP 1034276 A

**Non-patent literature cited in the description**

- **A. HADJIZADEH et al.** *Journal of Biomedical Nanotechnology,* 2013, vol. 9 (7), 1195